Europäisches Patentamt

(19) European Patent Office

Office européen des brevets

(11) Veröffentlichungsnummer : **0 341 594 B1**

(12) **EUROPÄISCHE PATENTSCHRIFT**

(45) Veröffentlichungstag der Patentschrift :
**22.01.92 Patentblatt 92/04**

(51) Int. Cl.⁵ : **C07C 65/11,** C07D 319/12

(21) Anmeldenummer : **89108119.2**

(22) Anmeldetag : **05.05.89**

(54) Verfahren zur Herstellung eines Addukts aus 2-Hydroxy-naphthalin-6-carbonsäure und 1,4-Dioxan und dessen Verwendung.

(30) Priorität : **10.05.88 DE 3815929**

(43) Veröffentlichungstag der Anmeldung :
**15.11.89 Patentblatt 89/46**

(45) Bekanntmachung des Hinweises auf die
Patenterteilung :
**22.01.92 Patentblatt 92/04**

(84) Benannte Vertragsstaaten :
**BE CH DE FR GB IT LI NL**

(56) Entgegenhaltungen :
**EP-A- 0 325 925**
**US-A- 4 393 191**

(56) Entgegenhaltungen :
**MIKROCHIM. ACTA, Band 6, 1969, Seiten
1208-1209, Univ. Innsbruck, AU; M. KUH-
NERT-BRANDSTAETTER et al.:
"Lösungsmitteleinschlüsse in Kristallen von
p-substituierten Benzoesäurederivaten"**

(73) Patentinhaber : **HOECHST
AKTIENGESELLSCHAFT
Postfach 80 03 20
W-6230 Frankfurt am Main 80 (DE)**

(72) Erfinder : **von Plessen, Helmold, Dr.
Kugelherrnstrasse 16
W-6240 Königstein/Taunus (DE)**
Erfinder : **Rittner, Siegbert, Dr.
Kornblumenweg 5
W-6082 Mörfelden-Walldorf (DE)**

**Beschreibung**

2-Hydroxy-naphthalin-6-carbonsäure (im folgenden auch kurz "2,6-Säure" genannt) wird technisch hergestellt nach einer modifizierten Kolbe-Schmitt-Reaktion. Dabei wird das Kaliumsalz des 2-Naphthols unter Druck bei 200-300°C mit Kohlendioxid umgesetzt (US-PS 4329494, EP-PS 53824, US-PS 1593816, US-PS 4287357, EP-PS 81753). Bei dieser Reaktion entstehen beträchtliche Anteile an Zersetzungsprodukten (Teere und Harze), die sich ebenso wie die entstehenden Nebenprodukte (2-Hydroxy-naphthalin-3-carbonsäure, 2-Hydroxy-naphthalin-3,6-dicarbonsäure) nur schwierig abtrennen lassen.

Bisher wird die Rohsäure nach Abtrennung von 2-Naphthol in aufwendiger Weise aufgearbeitet, und zwar durch Extraktion, Kohleadsorption und Umfällung aus Wasser

In der älteren EP-Anmeldung 89100236.2 (EP-OS 325925) ist ein Verfahren zur Reinigung von 2-Hydroxy-naphthalin-6-carbonsäure durch Umkristallisation beschrieben, das dadurch gekennzeichnet ist, daß man die rohe Säure aus mit Wasser mischbaren linearen oder cyclischen aliphatischen Äthern, aliphatischen Polyäthern und aliphatischen Hydroxyäthern oder aus mindestens 10 gew.-%igen wäßrigen Lösungen dieser Äther umkristallisiert ; nach den Ansprüchen 3 und 8 soll die Umkristallisation aus einer 20 bis 90 bzw. 35 bis 50 gew.-%igen wäßrigen Lösung von 1,4-Dioxan erfolgen und nach Anspruch 9 aus einer mindestens 90 gew.-%igen wäßrigen Lösung von 1,4-Dioxan oder aus wasserfreiem 1,4-Dioxan. Nach den Beispielen 3 und 5 soll aus einer Lösung von 2,6-Säure in einem Gemisch von Wasser und 1,4-Dioxan 2,6-Säure auskristallisieren, die nach Beispiel 3 noch einmal, und zwar aus 35 vol.-%igem Äthanol umkristallisiert wird, nach Beispiel 5 jedoch das Endprodukt darstellt. In Wirklichkeit handelt es sich bei dem aus dem Gemisch von Dioxan und Wasser ausfallenden Produkt jedoch um ein Adukt aus 2,6-Säure und 1,4-Dioxan.

Es wurde nun gefunden, daß man ein solches Adukt aus 2-Hydroxy-naphthalin-6-carbonsäure und 1,4-Dioxan auch dann erhalten kann, wenn man 2-Hydroxy-naphthalin-6-carbonsäure in Mischungen aus 1,4-Dioxan mit anderen organischen Lösungsmitteln als Äthern, und gegebenenfalls Wasser, löst und dann kristallisieren läßt. Dieses Adukt besteht gewöhnlich pro Mol aus etwa 2 Mol 2,6-Säure und 1 Mol Dioxan.

Ein weiterer Gegenstand ist daher ein Verfahren zur Herstellung eines Dioxan-Adukts der 2-Hydroxy-naphthalin-6-carbonsäure, das pro Mol aus 1 Mol 1,4-Dioxan und etwa 2 Mol 2-Hydroxy-naphthalin-6-carbonsäure besteht, das dadurch gekennzeichnet ist, daß man 2-Hydroxy-naphthalin-6-carbonsäure in Mischungen aus 1,4-Dioxan mit anderen organischen Lösungsmitteln als Äthern, und gegebenenfalls Wasser, löst und dann kristallisieren läßt.

Zur Herstellung des Adukts kann man technisch gewonnene 2,6-Säure direkt einsetzen.

Das Adukt kristallisiert aus dioxanhaltiger Lösung in Form wohl ausgebildeter stengeliger und tafeliger Kristalle, die sich durch Sedimentation, Filtration oder Zentrifugation von der Mutterlauge trennen lassen. Das Kristallisat wird beispielsweise abfiltriert und bei etwa 50°C unter vermindertem Druck im Stickstoffstrom getrocknet.

Eine Ausführungsform der Erfindung besteht darin, daß man eine Mischung von Dioxan mit Wasser und mit ihm mischbaren organischen Lösungsmitteln, die von Äthern verschieden sind, gleichzeitig als Mischungskomponenten verwendet ; beispielsweise kann man aus ternären Gemischen Dioxan/Wasser/niedere Alkohole das Dioxan-Adukt auskristallisieren lassen. Der Dioxangehalt der Mischungen mit organischen Lösungsmitteln und gegebenenfalls zusätzlich Wasser beträg im allgemeinen mindestens 10 Gew.-%, vorzugsweise mindestens 20 Gew.-%, insbesondere mindestens 30 Gew.-%.

Das Adukt läßt sich bei Zimmertemperatur unbegrenzt lagern. Will man daraus dioxanfreie 2,6-Säure zurückgewinnen, so genügt es, das Adukt auf etwa 100°C unter Vakuum zu erwärmen. Dabei wird das lose gebundene Dioxan praktisch vollständig abgespalten, und man erhält reine 2,6-Säure als amorphes weißes Pulver oder feinteiliges Granulat. Auch durch Umkristallisation des Adukts aus einem anderen Lösungsmittel, z.B. verdünntem Äthanol, läßt sich dioxanfreie 2,6-Säure zurückgewinnen. Ferner kann dioxanfreie 2,6-Säure durch Lösen des Adukts in Natriumhydroxid-Lösung und nachfolgende Fällung mit verdünnter Mineralsäure erhalten werden.

Seine besonderen Eigenschaften machen das erfindungsgemäße Adukt zu einem wertvollen Zwischenprodukt. So läßt sich 2-Hydroxy-naphthalin-6-carbonsäure, die bisher nur schwierig aus den Reaktionsprodukten der Kolbe-Schmitt-Reaktion rein zu gewinnen war, auf dem Weg über das Adukt in ausgezeichneter Reinheit herstellen. Die Möglichkeit, 2,6-Säure über das Adukt praktisch vollständig von Verunreinigungen wie 2-Hydroxy-naphthalin-3-carbonsäure, 2-Hydroxy-naphthalin-3,6-dicarbonsäure und 2-Naphthol zu befreien, die bei der Synthese als Nebenprodukte entstehen, bedeutet einen überraschenden Fortschritt gegenüber den üblichen umständlichen Verfahren zur Reinigung von Carbonsäuren. So können Carbonsäuren z.B. gemäß US-PS 2189726 durch mehrstufige Wäsche mit Wasser unter Erhitzen oder durch Wasserdampfdestillation gereinigt werden.

Das Adukt kann als Kupplungskomponente für Azofarbstoffe eingesetzt werden, wobei 1 Mol Adukt mit 2 Mol einer Diazokomponente umgesetzt wird.

Das bei der Reaktion frei werdende Dioxan kann zur Verdünnung und Homogenisierung des Reaktionsgemisches dienen.

Von besonderer Bedeutung ist das Addukt für die Gewinnung sehr reiner 2-Hydroxy-naphthalin-6-carbonsäure ("fibre grade" Qualität), die für die Polyveresterung mit organischen Hydroxycarbonsäuren, wie p-Hydroxy-benzoesäure, benötigt wird. Die so erhaltenen reinen Polyester sind wetvolle Ausgangsstoffe für die Herstellung von Kunststoffen oder Fasern (US-PS 4393191).

Beispiel

12,5 Teile rohe 2,6-Säure wurden in einem Gemisch von 42 Teilen Eisessig und 41 Teilen Dioxan unter Rühren und Erhitzen gelöst. Nach Vermischen mit 1 Teil Aktivkohle wurde 2 min lang gerührt und anschließend filtriert. Bei langsamem Abkühlen schied sich das kristalline Addukt aus der Lösung ab, das abfiltriert und mit 20 gew.-°%igem Äthanol gewaschen wurde. Trocknung unter vermindertem Druck über konzentrierter Schwefelsäure ergab 11, 3 Teile des Addukts.

**Patentansprüche**

1. Verfahren zur Herstellung eines Addukts aus 2-Hydroxy-naphthalin-6-carbonsäure und 1,4-Dioxan, dadurch gekennzeichnet, daß man 2-Hydroxy-naphthalin-6-carbonsäure in Mischungen aus 1,4-Dioxan mit anderen organischen Lösungsmitteln als Äthern, und gegebenenfalls Wasser, löst und dann kristallisieren läßt.

2. Verfahren zur Herstellung eines Dioxan-Addukts der 2-Hydroxy-naphthalin-6-carbonsäure, das pro Mol aus 1 Mol 1,4-Dioxan und etwa 2 Mol 2-Hydroxy-naphthalin-6-carbonsäure besteht, dadurch gekennzeichnet, daß man 2-Hydroxy-naphthalin-6-carbonsäure in Mischungen aus 1,4-Dioxan mit anderen organischen Lösungsmitteln als Äthern, und gegebenenfalls Wasser, löst und dann kristallisieren läßt.

3. Verwendung des nach dem Verfahren gemäß Anspruch 1 oder 2 erhaltenen Dioxan-Addukts, dadurch gekennzeichnet, daß man das Addukt durch thermische Behandlung in 2-Hydroxy-naphthalin-6-carbonsäure umwandelt und diese dann als Monomeres für Kunststoffe oder Fasern verwendet.

**Claims**

1. A process for the preparation from an adduct of 2-hydroxynaphthalene-6-carboxylic acid with 1,4-dioxane, which comprises dissolving 2-hydroxynaphthalene-6-carboxylic acid in a mixture of 1,4-dioxane with organic solvents other than ethers, and, if desired, water and then allowing it to crystallize.

2. A process for the preparation of a dioxane adduct of 2-hydroxynaphthalene-6-carboxylic acid which comprises 1 mol of 1,4-dioxane and about 2 mol of 2-hydroxynaphthalene-6-carboxylic acid per mol, wherein 2-hydroxynaphthalene-6-carboxylic acid is dissolved in a mixture of 1,4-dioxane with organic solvents other than ethers and, if desired, water and then allowed to crystallize.

3. Use of the dioxane adduct obtained by the process as claimed in claim 1 or 2, wherein the adduct is converted by a heat treatment to 2-hydroxy-naphthalene-6-carboxylic acid, which acid is used as a monomer for plastics or fibers.

**Revendications**

1. Procédé pour préparer un adduit à partir de l'acide hydroxy-2 naphtalène-carboxylique-6 et du dioxanne-1,4, procédé caractérisé en ce qu'on dissout l'acide hydroxy-2 naphtalène-carboxylique-6 dans des mélanges de dioxanne-1,4 avec des solvants organiques autres que des éthers, et éventuellement d'eau, puis on fait cristalliser.

2. Procédé pour préparer un adduit qui dérive du dioxanne et de l'acide hydroxy-2 naphtalène-carboxylique-6 et qui est constitué de 1 mol de dioxanne 1,4 et d'environ 2 mol d'acide hydroxy-2 naphtalène-carboxylique-6 par mole, procédé caractérisé en ce qu'on dissout l'acide hydroxy-2 naphtalène-carboxylique-6 dans des mélanges de dioxanne-1,4 avec des solvants organiques autres que des éthers, et éventuellement d'eau, puis on fait cristalliser.

3. Application de l'adduit de dioxanne obtenu par le procédé de la revendication 1 ou de la revendication 2, application caractérisée en ce qu'on transforme l'adduit, par un traitement à la chaleur, en l'acide hydroxy-2 naphtalène-carboxylique-6, puis on utilise celui-ci comme monomère pour des matières plastiques ou des fibres.